# EUROPEAN PATENT APPLICATION

(11) **EP 2 674 179 A1**
(43) Date of publication of application: **18.12.2013**
(21) Application number: 12004543.0
(22) Date of filing: 15.06.2012
(51) Int. Cl.: A61M 5/20, A61M 5/24

(54) **Injection device**

(71) Applicant: Ares Trading S.A., 1170 Aubonne (CH)
(72) Inventor: Wurmbauer, Werner, 9063 Maria Saal (AT); Schopf, Josef, 8753 Aichdorf/Fohnsdorf (AT); Schatz, Bernhard, 9020 Klagenfurt (AT)
(74) Representative: Micheli & Cie SA

(57) **Abstract**

An injection device for injecting liquid medicine to a patient comprises a main part (13) and a removable module (14), wherein the main part is arranged to house a medicine container (11) connected to a needle (9) and the removable module comprises a skin contact surface (10), the skin contact surface having a through hole (8) for passage of the needle and being intended to rest on the patient's skin during the injection.

## Description

The present invention relates to an injection device for injecting liquid medicine to a patient.

One example of such a device is described in WO 2005/077441. In this device, the skin contact surface, i.e. the surface intended to contact the area of the patient's skin where injection is to be performed, has a through hole for passage of a needle connected to a medicine container. After use of the device, the skin contact surface and the through hole may be contaminated by the medicine and/or by pathogenic agents from the patient. A careful cleaning and/or disinfection and/or sterilisation may thus be required before any subsequent use of the device, especially if the device is to be used by another patient. Such a cleaning operation is not an easy task.

The present invention aims at remedying or at least attenuating this drawback and, to this effect, provides an injection device for injecting liquid medicine to a patient, comprising a main part and a removable module, wherein the main part is arranged to house a medicine container connected to a needle and the removable module comprises a skin contact surface, said skin contact surface having a through hole for passage of the needle and being intended to rest on the patient's skin during the injection.

Thus, in the present invention the skin contact surface is defined by a module that can be removed from the device for facilitating the cleaning and/or disinfection and/or sterilisation operations.

The injection device according to the invention may also comprise a mechanism for moving the medicine container toward the skin contact surface from a first position to a second position or away from the skin contact surface from the second position to the first position.

The removable module may also comprise one or more of the following elements:
- a sensor for detecting contact between the skin contact surface and the patient's skin,
- releasable tabs for holding a needle assembly including the needle in the through hole while the medicine container is moved from the first position to the second position in order to automatically connect the needle to the medicine container;
- a sensor for detecting insertion of the needle assembly into the through hole;
- one or more abutments for limiting insertion of the needle assembly into the through hole:
- a retaining member which may be actuated to retain the needle in the through hole while the medicine container is moved from the second position to the first position in order to automatically disconnect the needle from the medicine container;
- an engine for actuating the retaining member;
- electrical contacts cooperating with corresponding electrical contacts of the main part.

The injection device may further comprise a button which, in a rest position, locks the removable module to prevent its detachment from the main part and which, when actuated, unlocks the removable module to allow its detachment from the main part.

The button may be concealed by a removable battery pack of the injection device.

Other features and advantages of the present invention will be clearly apparent upon reading the following detailed description made with reference to the appended drawings in which:
- figure 1 is a perspective view of the outside of an injection device according to the invention;
- figure 2 is the same perspective view as in figure 1 but showing a removable module detached from a main part of the device;
- figures 3 and 4 are perspective views from above and below, respectively, of the removable module;
- figure 5 is another perspective view from above of the removable module;
- figure 6 is a plan view showing a mechanism provided in the removable module;
- figure 7 shows different steps of a needle attachment sequence performed with the injection device according to the invention;
- figure 8 shows different steps of a needle detachment sequence performed with the injection device according to the invention; in this figure, as in figure 7, the main part of the device to which the removable module is attached has not been represented;
- figure 9 shows different steps of a sequence for removing the removable module from the injection device;
- figure 10 shows different steps of a sequence for (re)attaching the removable module to the main part of the injection device.

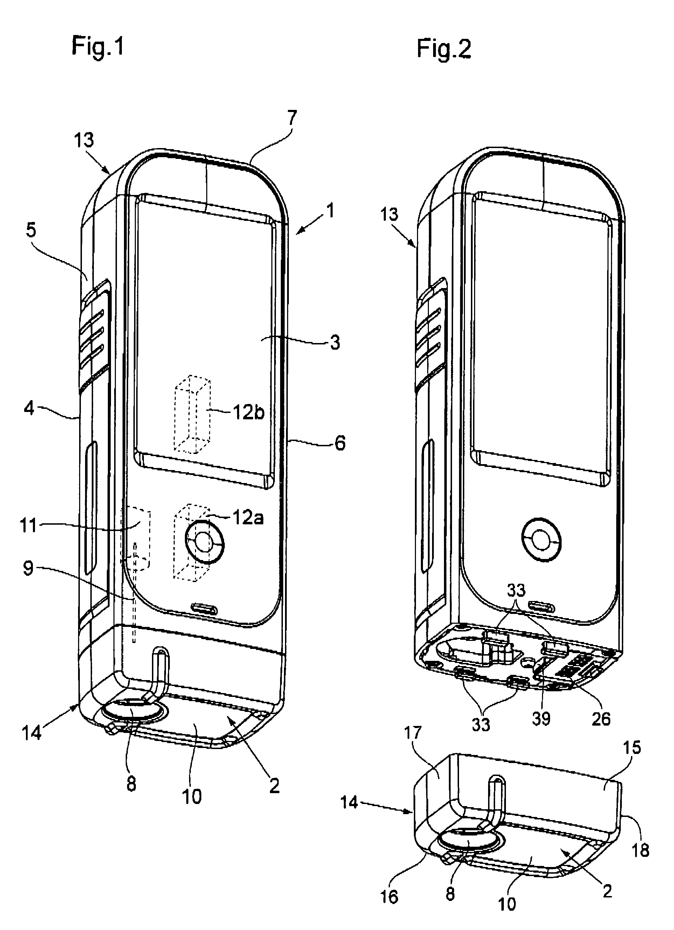

Figure 1 shows the housing 1 of a medicine injection device according to the invention. The device according to the invention is of the type described in WO 2005/077441. The housing 1 has a base wall 2, a front wall 3, a rear wall 4 opposite the front wall 3, opposite side walls 5, 6 and a top wall 7 opposite the base wall 2. The base wall 2 comprises a through hole 8 permitting passage of a needle 9. An outer surface 10 of the base wall 2 constitutes a skin contact surface which is traversed by the through hole 8 and which, in use, is intended to be applied on the area of the patient's skin where injection is to be performed. At the base wall 2, more precisely on the inner surface of the base wall 2, is provided a skin sensor for detecting contact between the skin contact surface 10 and the patient's skin. The skin sensor may be a capacitive sensor as described in WO 2007/088444.

The needle 9 is connected to a medicine container 11 inside the housing 1. A first electromechanical actuator assembly 12a, represented only diagrammatically in figure 1, is also provided inside the housing 1 for moving axially the medicine container 11 (more exactly a structure holding the medicine container 11) with its needle 9 so that these elements 9, 11 can take a retracted position, fully inside the housing 1, and an operating position in which the needle 9 protrudes from the through hole 8 to pierce the patient's skin. A second electromechanical actuator assembly 12b is arranged to drive a piston in the medicine container 11. The electromechanical actuator assemblies 12a, 12b are controlled by a control unit inside the housing 1 according to a sequence comprising a first step in which the medicine container 11 is moved from its retracted position to its operating position, a second step in which the piston is moved to perform the injection into the patient and a third step in which the medicine container 11 is moved back to its retracted position after the injection of a determined dose has been performed. The said sequence is triggered by the control unit only after the skin sensor has detected that the injection device is placed on the patient's skin, i.e. that the skin contact surface 10 rests on the patient's skin, and after an injection button has been pressed by the patient.

The needle 9 is automatically connectable to the medicine container 11. More precisely, to connect the needle 9 to the medicine container 11 the user inserts a needle assembly into the through hole 8 between releasable tabs which will hold the needle assembly in the through hole 8. The needle assembly includes the needle, a needle hub supporting the needle and a needle cap covering the needle and the needle hub. Insertion of the needle assembly between the releasable tabs is detected by a sensor, which triggers an axial motion of the medicine container 11 toward the base wall 2 from its retracted position to a position where the needle and the needle hub are connected to the medicine container 11. Next, the motion of the medicine container 11 is reversed and the medicine container 11 with the needle 9 and the needle hub moves back to its retracted position while the needle cap is blocked by one or more abutments in the through hole 8. The user then manually removes the needle cap from the through hole 8.

The needle 9 is also automatically detachable from the medicine container 11. To detach the needle 9 from the medicine container 11, the user inserts the empty needle cap into the through hole 8 between the releasable tabs. This insertion is detected by the aforementioned sensor and causes the medicine container 11 with the needle 9 to move toward the base wall 2 until the needle 9 and its needle hub are inside the needle cap. Then the user may actuate a needle release button which will move a retaining member transversely in the through hole 8 so as to block the needle hub and, with it, the needle 9 in the direction from the through hole 8 to the retracted position of the medicine container 11. Thereafter, a reverse motion is imparted to the medicine container 11 which moves back to its retracted position while the needle hub and, with it, the needle 9 are retained in the through hole 8. The user can then disengage the needle assembly, including the needle cap, the needle hub and the needle 9, from the through hole 8 and its releasable tabs.

More details concerning the structure and operation of the injection device may be found in WO 2005/077441 and WO 2007/088444 which are herein incorporated by reference.

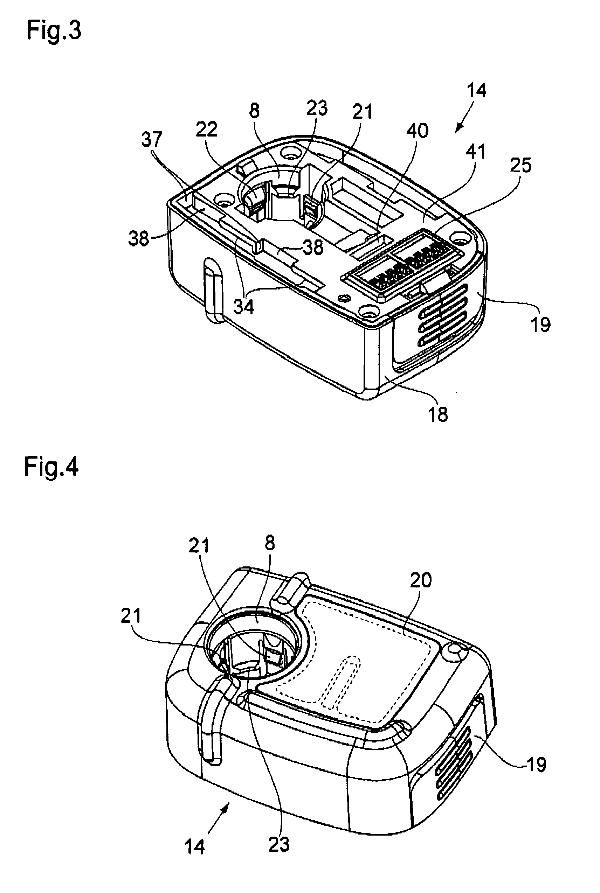

According to the present invention, the injection device is in two parts assembled together, i.e. a main part 13 and a base cover module 14. These two parts 13, 14 are detachable from one another, as shown in figure 2. In other words the base cover module 14 is removable from the injection device. The main part 13 includes the main components of the device, in particular the medicine container 11, the electromechanical actuator assemblies 12a, 12b and the control unit. The base cover module 14 defines the entire base wall 2 of the housing 1, with its through hole 8 and its skin contact surface 10, and also comprises a front wall 15, a rear wall 16 and side walls 17, 18 respectively forming part of the front wall 3, rear wall 4 and side walls 5, 6 of the housing 1. A slide button 19 (see figure 3) is located in the side wall 18. As shown in figures 3 to 5, the base cover module 14 further comprises the aforementioned skin sensor, designated by the reference numeral 20, the aforementioned releasable tabs, designated by 21, the aforementioned sensor, designated by 22, for detecting insertion of the needle assembly into the through hole 8, the aforementioned abutments, designated by 23, for limiting the insertion of the needle assembly into the through hole 8 and the aforementioned retaining member, designated by 24, for retaining the needle hub and the needle attached to it in the through hole 8 while the medicine container is moved back to its retracted position for detachment of the needle. The base cover module 14 also has electrical contacts 25 which cooperate with corresponding electrical contacts 26 (see figure 2) of the device main part 13 for passage of electricity between the two parts 13, 14.

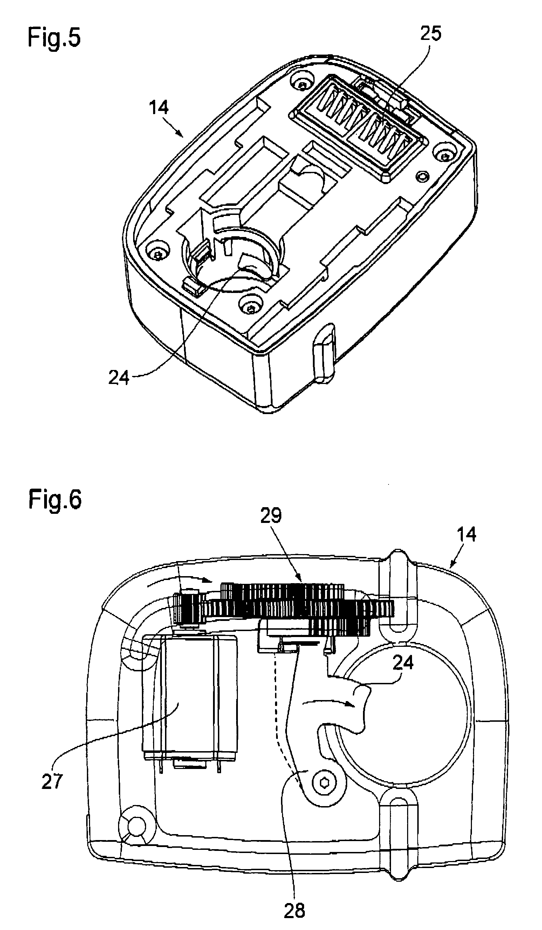

In the injection device described in WO 2005/077441 the retaining member is driven directly by the force exerted by the user on a mechanical button. In the present invention, on the other hand, the retaining member 24 is driven by an electric engine 27 provided in the base cover module 14. As can be seen in figure 6, the electrical engine 27 is arranged to drive a lever 28 via a gearbox 29, which lever 28 defines the retaining member 24.

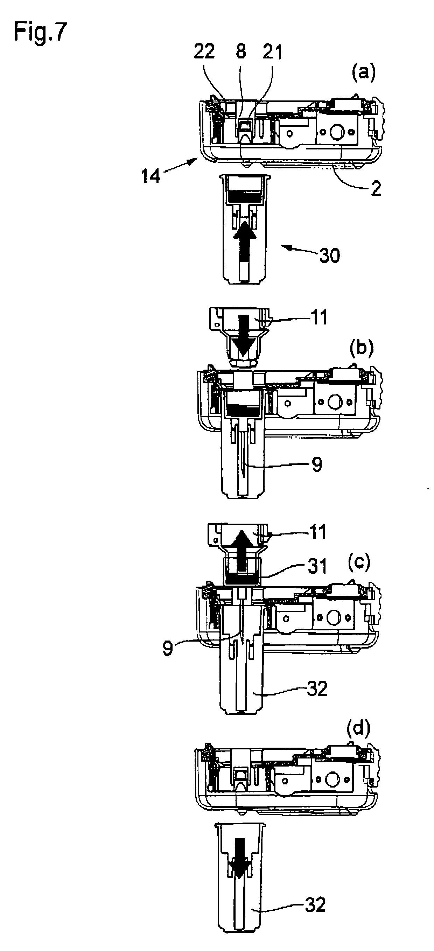

Figure 7 shows the needle attachment sequence. In a first step (figure 7(a)), the needle assembly 30 is inserted by the user into the through hole 8, between the releasable tabs 21. In a second step (figure 7(b)), the sensor 22 detects the presence of the needle assembly 30 and the medicine container 11 is moved from its retracted position toward the base wall 2 until it is connected to the needle 9 included in the needle assembly 30. In a third step (figure 7(c)), the medicine container 11 is moved back to its retracted position, with the needle hub 31 and needle 9 attached to it, while the needle cap 32 is retained by the abutments 23. In a fourth step (figure 7(d)), the user removes the needle cap 32 from the through hole 8.

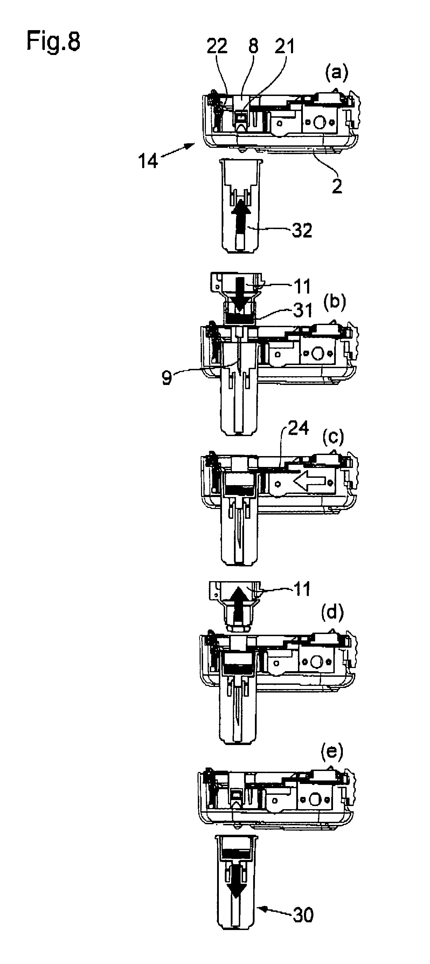

Figure 8 shows the needle detachment sequence. In a first step (figure 8(a)), the empty needle cap 32 is inserted by the user into the through hole 8, between the releasable tabs 21. In a second step (figure 8(b)), the sensor 22 detects the needle cap 32 and the medicine container 11 with the needle hub 31 and needle 9 is moved from its retracted position toward the base wall 2 until the needle hub 31 and needle 9 are inside the needle cap 32 (figure 8(c)). In a third step (figure 8(c)), the engine 27 is actuated automatically so that the lever 28 defining the retaining member 24 is moved transversely toward the inside of the through hole 8 so as to prevent the needle hub 31 from moving toward the medicine container's retracted position. In a fourth step (figure 8(d)), the medicine container 11 is moved back to its retracted position without the needle hub 31, which is retained by the retaining member 24, and without the needle 9, which is fixed to the needle hub 31. In a fifth step (figure 8(e)), the user removes the needle assembly 30 from the through hole 8.

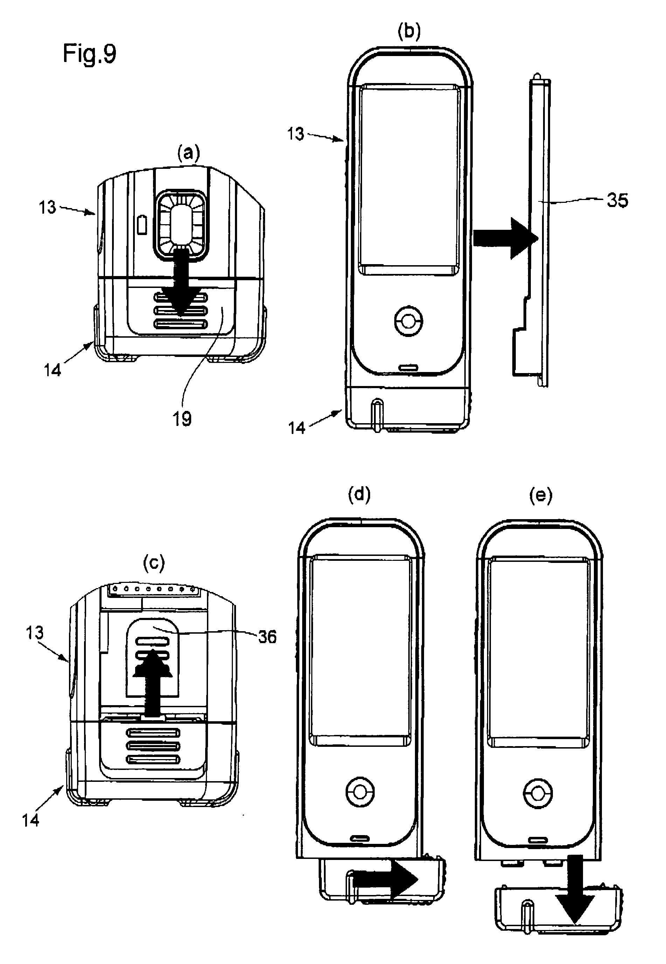

Figure 9 shows how the base cover module 14 may be detached from the device main part 13. The base cover module 14 and the main part 13 are assembled by hooked tabs 33 (see figure 2) provided on one of the parts 13, 14, for example the main part 13, cooperating with corresponding recesses 34 (see figure 3) provided on the other of the parts 13, 14. In a first step (figure 9(a)) the slide button 19 is actuated so as to release a battery pack 35 provided in the side wall 6 of the device main part 13. The battery pack 35 is removed (figure 9(b)) and a slide button 36 (figure 9(c)) which was concealed by the battery pack 35 is actuated so as to unlock the base cover module 14. The base cover module 14 can then be moved laterally (figure 9(d)) up to a position defined by stops 37 (see figure 3) abutting against the tabs 33 (figure 2). In this position the tabs 33 are within open parts 38 of the recesses 34 and no longer prevent axial separation of the parts 13, 14 from one another. The base cover module 14 can thus be separated from the device main part 13 in the axial direction (figure 9(e)).

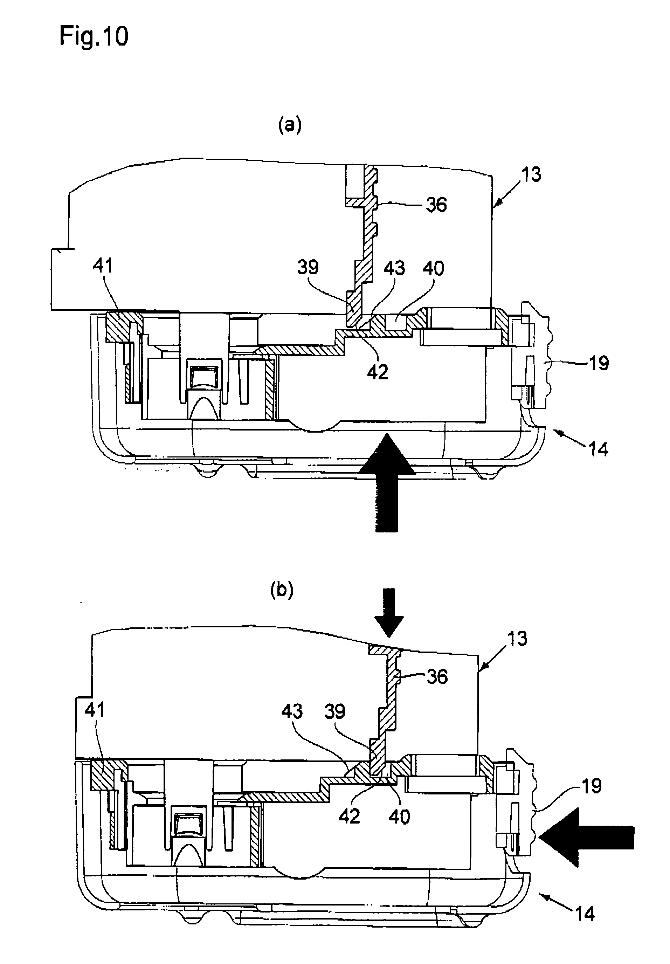

To attach the base cover module 14 to the device main part 13, the base cover module 14 is subjected to movements reverse to those shown in figures 9(e) and 9(d) and the battery pack 35 is reinserted. Figure 10 shows the operation of attaching the base cover module 14 to the device main part 13. The button 36 is spring-loaded and has an end portion 39 (also visible in figure 2) which is engaged in a hole 40 (also visible in figure 3) of the base cover module 14 in the attached position of the latter. The hole 40 is defined by a plate 41 which forms an upper wall for the base cover module 14 and which also defines the recesses 34 and is traversed by the through hole 8. Actuating the button 36 as shown in figure 9(c), against the action of its spring, retracts the button 36 out of the hole 40 and thus unlocks the base cover module 14. Upon attachment of the base cover module 14 to the device main part 13, more precisely upon the movement reverse to that shown in figure 9(d), an inclined surface 42 of the button end portion 39 slides on a corresponding inclined surface 43 of the plate 41, causing the button 36 to retract against the action of its spring until it is aligned with the hole 40 and thus pushed by the said spring into the hole 40 to lock the base cover module 14 (figure 10(b)). During this movement, a click is produced by the button end portion 39 engaging the hole 40, which click indicates to the user that the base cover module 14 is correctly attached to the device main part 13.

Thus, in the present invention the skin contact surface 10 intended to contact the injection site is defined by the base cover module 14 which may be removed from the injection device for cleaning and/or disinfection and/or sterilisation. The base cover module 14 may also be replaced by another identical module for the same or another patient. Such cleanability and interchangeability of the base cover module 14 allow the main part 13 of the device to be reused by another patient.

Moreover, the fact that the button 36 for unlocking the base cover module 14 is concealed by the battery pack 35 increases the security of use of the device since the base cover module 14 cannot be removed inadvertently.

## Claims

1. Injection device for injecting liquid medicine to a patient, comprising a main part and a removable module, wherein the main part is arranged to house a medicine container connected to a needle and the removable module comprises a skin contact surface, said skin contact surface having a through hole for passage of the needle and being intended to rest on the patient's skin during the injection.

2. Injection device according to claim 1, wherein the removable module comprises a sensor for detecting contact between the skin contact surface and the patient's skin.

3. Injection device according to claim 1 or 2, further comprising a mechanism for moving the medicine container toward the skin contact surface from a first position to a second position or away from the skin contact surface from the second position to the first position.

4. Injection device according to claim 3, wherein the removable module comprises releasable tabs for holding a needle assembly including the needle in the through hole while the medicine container is moved from the first position to the second position in order to automatically connect the needle to the medicine container.

5. Injection device according to claim 4, wherein the removable module comprises a sensor for detecting insertion of the needle assembly into the through hole.

6. Injection device according to claim 4 or 5, wherein the removable module comprises one or more abutments for limiting insertion of the needle assembly into the through hole,

7. Injection device according to any one of claims 3 to 6, wherein the removable module comprises a retaining member which may be actuated to retain the needle in the through hole while the medicine container is moved from the second position to the first position in order to automatically disconnect the needle from the medicine container.

8. Injection device according to claim 7, wherein the removable module comprises an engine for actuating the retaining member.

9. Injection device according to any one of claims 1 to 8, wherein the removable module comprises electrical contacts cooperating with corresponding electrical contacts of the main part.

10. Injection device according to any one of claims 1 to 9, further comprising a button which, in a rest position, locks the removable module to prevent its detachment from the main part and which, when actuated, unlocks the removable module to allow its detachment from the main part.

11. Injection device according to claim 10, wherein the button is concealed by a removable battery pack of the injection device.
